# EUROPEAN PATENT APPLICATION

(11) **EP 4 606 396 A1**
(43) Date of publication of application: **27.08.2025**
(21) Application number: 23888118.9
(22) Date of filing: 10.11.2023
(51) Int. Cl.: A61L 29/08, A61L 29/16

(54) **MEDICAMENT-COATED BALLOON COATING SOLUTION, COATING MATERIAL, MEDICAMENT-COATED BALLOON, PREPARATION METHOD THEREFOR, AND USE THEREOF**

(30) Priority: 11.11.2022 CN 202211413746
(71) Applicant: Cardio Navi MedTech (Wuhan) Co., Ltd., Wuhan, Hubei 430000 (CN)
(72) Inventor: XIONG, Dan, Wuhan, Hubei 430000 (CN); LI, Shuangshuang, Wuhan, Hubei 430000 (CN); LV, Meng, Wuhan, Hubei 430000 (CN); LI, Chao, Wuhan, Hubei 430000 (CN); ZHANG, Ying, Wuhan, Hubei 430000 (CN)
(74) Representative: Altmann Stößel Dick Patentanwälte PartG mbB
(86) International application number: PCT/CN2023/130996
(87) International publication number: WO 2024/099433

(57) **Abstract**

Provided are a medicament-coated balloon coating solution, a coating material, a medicament-coated balloon, a preparation method therefor, and use thereof. The medicament-coated balloon coating solution comprises an aqueous-phase solvent. The coating solution further comprises a plurality of core-shell structures formed by wrapping a medicament in a phospholipid bilayer. The core-shell structures are dispersed in the aqueous-phase solvent. A core of each core-shell structure comprises a plurality of particles loaded with the medicament. A shell of each core-shell structure is the phospholipid bilayer. The phospholipid bilayer comprises a hydrophilic group in an outer layer and a hydrophobic group in an inner layer. The plurality of particles loaded with the medicament comprise a plurality of nanocrystalline particles comprising the medicament. Nanocrystalline is combined with liposome, such that advantages of two types of medicament carriers are combined. The formed phospholipid bilayer increases the solubility of a poorly soluble medicament on the medicament-coated balloon, the medicament loading capacity is high, the stability of the medicament carriers is good, and the medicament crystal form is stable, such that the medicament release rate is controllable.

## Description

### Technical Field

The present invention relates to the field of interventional treatment devices, in particular to a coating material of drug-coated balloon, a drug-coated balloon, a coating solution for the drug-coated balloon and a preparation method therefor, and a use thereof.

### Background Art

Nanocrystals can effectively increase the solubility of poorly soluble drugs and possess a high drug loading capacity, theoretically up to 100%. However, Ostwald ripening leads to system instability. Over extended storage periods, nanocrystals gradually re-deposit into larger particles, resulting in unstable particle sizes and physical properties during storage. Due to their high surface energy and rapid dissolution rate, nanocrystals become increasingly unstable in complex in vivo environments due to surface erosion and progressive loss of spatial stability. Additionally, drug-coated balloons composed solely of nanocrystals may experience uncontrolled dissolution or drug release in vivo. Liposomes offer controlled drug release capabilities. While nanocarriers exhibit good biocompatibility and stability and can rapidly access tissues, the use of poorly soluble drugs is limited by their retention within phospholipid bilayers, as the drug loading capacity of liposomes is low. Nanocrystal-liposome complexes combine the advantages of both, achieving high drug loading capacity, enhanced stability, and controllable drug release rates.

### Summary of the Invention

An embodiment of the present invention provides a coating solution for a drug-coated balloon. The coating solution comprises an aqueous solvent and further includes a plurality of core-shell structures formed by encapsulating drugs within a phospholipid bilayer. These core-shell structures are dispersed in the aqueous solvent. Each core-shell structure comprises a core consisting of a plurality of drug-loaded particles and a shell formed by the phospholipid bilayer. The phospholipid bilayer comprises hydrophilic groups on the outer layer and hydrophobic groups on the inner layer. The plurality of drug-loaded particles consist of a plurality of nanocrystals particles containing the drug.

Preferably, the particle size of the core-shell structures is between 200 and 900 nm.

Preferably, the particle size d₅₀ of the nanocrystals particles is between 150 and 900 nm; wherein in the preparation process of the nanocrystals particles, a surfactant is utilized.

Preferably, the surfactant is selected from one or more of the following: vitamin E polyethylene glycol succinate, Poloxamer 188, Poloxamer 407, Tyloxapol, docusate sodium, polyoxyl 15-hydroxystearate, polyoxyethylene (40) hydrogenated castor oil, polyoxyethylene (60) hydrogenated castor oil, polyoxyethylene (60) castor oil, polyoxyethylene (40) castor oil, polyoxyethylene (35) castor oil, polyoxyethylene (20) castor oil, polyoxyethylene (10) castor oil, polyethylene glycol hexadecyl octadecyl ether 20, polyethylene glycol hexadecyl octadecyl ether 12, polyoxyethylene monocetyl ether (Ceteth-10), polyoxyethylene (10) lauryl ether (Brij 56), polyoxyethylene (20) lauryl ether (Brij 58), polyoxyethylene (23) lauryl ether (Brij 35), polyoxyethylene (2) lauryl ether (Brij 52), polyoxyethylene (25) oleate (Myrj 49), polyoxyethylene (40) palmitate (Myrj 52), sodium dodecyl sulfate, Tween 80, Tween 60, Tween 40, propylene glycol monolaurate, polyoxyethylene (20) lauryl ether, and polyoxyethylene monocetyl ether.
Preferably, the drugs include Sirolimus, Zotarolimus and Everolimus, Tacrolimus, Temsirolimus, Pimecrolimus, Deforolimus, and Ridaforolimus;
Preferably, the raw materials for preparing the phospholipid bilayer include phospholipids and cholesterols;
Preferably, the phospholipids include one or more of yolk lecithin, soybean phospholipid, hydrogenated yolk lecithin, hydrogenated soybean phospholipid, cephalin, phosphatidylethanolamine, dimyristyl phosphatidylcholine (DMPC), stearamide (SA), sunflower phospholipid, 1,2-diformyl-sn-glycero-3-phosphocholine, 1,2-dipalmitoyl-sn-glycero-3-phosphocholine, 1,2-distearoyl-sn-glycero-3-phosphocholine, 1,2-dioleoyl-sn-glycero-3-phosphocholine, 1,2-dierucoyl-sn-glycero-3-phosphocholine, 1-dipalmitoyl-dioleoyl-sn-glycero-3-phosphocholine, 1,2-diformyl-sn-glycero-3-phospho-rac-glycerol sodium salt, 1,2-dipalmitoyl-sn-glycero-3-phospho-rac-glycerol sodium salt, 1,2-distearoyl-sn-glycero-3-phospho-rac-glycerol sodium salt, 1,2-dioleoyl-sn-glycero-3-phospho-rac-glycerol sodium salt, 1-dipalmitoyl-dioleoyl-sn-glycero-3-phospho-rac-glycerol sodium salt, 1,2-diformyl-sn-glycero-3-phosphoethanolamine, 1,2-dipalmitoyl-sn-glycero-3-phosphoethanolamine, 1,2- distearoyl-sn-glycero-3-phosphoethanolamine, 1,2-dioleoyl-sn-glycero-3-phosphoethanolamine, 1,2-dioleoyl-sn-glycero-3-phospho-L-serine sodium salt, 1,2-dipalmitoyl-sn-glycero-3-phosphate monosodium salt, N-(carbonyl-methoxypolyethylene glycol-5000)-1,2-dipalmitoyl-sn-glycero-3-phosphoethanolamine sodium salt, N-(carbonyl-methoxypolyethylene glycol-2000)-1,2-distearoyl-sn-glycero-4-phosphoethanolamine sodium salt, and 1,2-dioleoyl-3-trimethylpropanyl ammonium chloride;
The cholesterols include cholesterol and DC cholesterol;
Preferably, the mass ratio of phospholipids to cholesterols is between 40:1 and 1:2, and more preferably between 30:1 and 1:1.

The present invention also provides a coating material for the drug-coated balloon, the coating material comprises a plurality of core-shell structures formed by wrapping drugs in a phospholipid bilayer. Wherein, a core of the core-shell structure comprises a plurality of particles loaded with drugs. A shell of the core-shell structure is the phospholipid bilayer. The phospholipid bilayer comprises a hydrophilic group in an outer layer and a hydrophobic group in an inner layer. The plurality of particles loaded with the drug comprise a plurality of nanocrystals particles comprising drugs.
Preferably, the particle size of the core-shell structures is 200 to 900 nm;
Preferably, the particle size d₅₀ of the nanocrystals particles is 150 to 900 nm; in the preparation process of nanocrystals particles, a surfactant is used;
Preferably, the surfactant is selected from one or more of vitamin E polyethylene glycol succinate, Poloxamer 188, Poloxamer 407, Tyloxapol, docusate sodium, polyoxyl 15-hydroxystearate, polyoxyethylene (40) hydrogenated castor oil, polyoxyethylene (60) hydrogenated castor oil, polyoxyethylene (60) castor oil, polyoxyethylene (40) castor oil, polyoxyethylene (35) castor oil, polyoxyethylene (20) castor oil, polyoxyethylene (10) castor oil, Polyethylene glycol hexadecyl octadecyl ether 20, polyethylene glycol hexadecyl octadecyl ether 12, polyoxyethylene monocetyl ether (Ceteth-10), polyoxyethylene (10) lauryl ether (Brij 56), polyoxyethylene (20) lauryl ether (Brij 58), polyoxyethylene (23) lauryl ether (Brij 35), polyoxyethylene (2) lauryl ether (Brij 52), polyoxyethylene (25) oleate (Myrj 49), polyoxyethylene (40) palmitate (Myrj 52) , sodium dodecyl sulfate, Tween 80, Tween 60, Tween 40, propylene glycol monolaurate, polyoxyethylene (20) lauryl ether, and polyoxyethylene monocetyl ether;
Preferably, the drugs include Sirolimus, Zotarolimus and Everolimus, Tacrolimus, Temsirolimus, Pimecrolimus, Deforolimus, and Ridaforolimus;
Preferably, the raw materials for preparing the phospholipid bilayer include phospholipids and cholesterols;
Preferably, the phospholipids include one or more of yolk lecithin, soybean phospholipid, hydrogenated yolk lecithin, hydrogenated soybean phospholipid, cephalin, phosphatidylethanolamine, dimyristyl phosphatidylcholine (DMPC), stearamide (SA), sunflower phospholipid, 1,2-diformyl-sn-glycero-3-phosphocholine, 1,2-dipalmitoyl-sn-glycero-3-phosphocholine, 1,2-distearoyl-sn-glycero-3-phosphocholine, 1,2-dioleoyl-sn-glycero-3-phosphocholine, 1,2-dierucoyl-sn-glycero-3-phosphocholine, 1-dipalmitoyl-dioleoyl-sn-glycero-3-phosphocholine, 1,2-diformyl-sn-glycero-3-phospho-rac-glycerol sodium salt, 1,2-dipalmitoyl-sn-glycero-3-phospho-rac-glycerol sodium salt, 1,2-distearoyl-sn-glycero-3-phospho-rac-glycerol sodium salt, 1,2-dioleoyl-sn-glycero-3-phospho-rac-glycerol sodium salt, 1-dipalmitoyl-dioleoyl-sn-glycero-3-phospho-rac-glycerol sodium salt, 1,2-diformyl-sn-glycero-3-phosphoethanolamine, 1,2-dipalmitoyl-sn-glycero-3-phosphoethanolamine, 1,2- distearoyl-sn-glycero-3-phosphoethanolamine, 1,2-dioleoyl-sn-glycero-3-phosphoethanolamine, 1,2-dioleoyl-sn-glycero-3-phospho-L-serine sodium salt, 1,2-dipalmitoyl-sn-glycero-3-phosphate monosodium salt, N-(carbonyl-methoxypolyethylene glycol-5000)-1,2-dipalmitoyl-sn-glycero-3-phosphoethanolamine sodium salt, N-(carbonyl-methoxypolyethylene glycol-2000)-1,2-distearoyl-sn-glycero-4-phosphoethanolamine sodium salt, and 1,2-dioleoyl-3-trimethylpropanyl ammonium chloride; the cholesterols include cholesterol and DC cholesterol;
Preferably, the mass ratio of phospholipids to cholesterols is between 40:1 and 1:2, and more preferably between 30: 1 and 1:1.

The present invention also provides a drug-coated balloon, wherein the surface of the drug-coated balloon is coated with any of the above-mentioned materials.

The present invention also provides a use of the drug-coated balloon in the treatment of atherosclerosis, stenosis and/or restenosis of coronary arteries, peripheral vessels, or intracranial arteries.

The present invention also provides a preparation method of the coating solution for drug-coated balloon. The method comprises the following steps:
(1) Preparing a plurality of nanocrystals particles comprising drugs; the method for preparing the nanocrystals particles comprises: anti-solvent method, high pressure homogenization or microfluidic method;
(2) Accurately weigh cholesterols, phospholipids and solvents in aqueous phase , respectively, and obtain a milky white solution after magnetic stirring;
(3) Add nanocrystals particles to the milky white solution, mix well by magnetic stirring to obtain the coating solution for drug-coated balloon;

Wherein, the coating solution comprises an aqueous solvent, and the coating solution further comprises a plurality of core-shell structures formed by wrapping drugs in a phospholipid bilayer. The core-shell structures are dispersed in the solvent in aqueous phase. A core of the core-shell structure comprises a plurality of particles loaded with the drug. A shell of the core-shell structure is the phospholipid bilayer. The phospholipid bilayer comprises a hydrophilic group in an outer layer and a hydrophobic group in an inner layer.
Preferably, after Step (3), there is Step (4) for adding a thickener to the above-mentioned solution, and mixing well by magnetic stirring to obtain the coating solution for drug-coated balloon;
Preferably, the thickener comprises sodium alginate;
Preferably, in Step (2), after magnetic stirring, 5 to 10 times of homogenization are performed by a high pressure homogenizer or a microfluidic device to obtain a milky white solution;
Preferably, the drugs in Step S1 include Sirolimus, and the organic solvents include methanol;
Preferably, the mass ratio of Sirolimus to sodium dodecyl sulfate is between 5:1 and 1:2;
Preferably, the anti-solvent method comprises the following steps:
   S1. Accurately weigh Sirolimus and organic solvent, respectively, and stir magnetically until a transparent and uniform phase A solution is obtained;
   S2. Accurately weigh sodium dodecyl sulfate and solvent in aqueous phase, respectively, and stir magnetically until a transparent and uniform phase B solution is obtained;
   S3. Add phase A solution to phase B solution quickly, and stir magnetically to obtain a nanocrystals suspension;
   S4. Add water to the suspension and mix well, and then freeze-dry to obtain nanocrystals particles;
Preferably, the particle size d₅₀ of the nanocrystals particles is 150 to 900 nm, and the mass ratio of Sirolimus to sodium dodecyl sulfate is between 5:1 and 1:2;
Preferably, the high pressure homogenization method comprises the following steps:
   S1. Accurately weigh Sirolimus, solvent in aqueous phase and sodium dodecyl sulfate, respectively, and stir magnetically to obtain a suspension;
   S2. Perform several times of homogenization to the suspension through a high pressure homogenizer to obtain a nanocrystals suspension;
   S3. Freeze-dry to obtain Sirolimus nanocrystals;
Preferably, the particle size d₅₀ of the nanocrystals particles is 150 to 900 nm, the homogenization pressure is 1,000 to 2,000 bar, the homogenization are performed for 5 to 10 times, and the mass ratio of Sirolimus to sodium dodecyl sulfate is between 5:1 and 1:2;
Preferably, the microfluidic method comprises the following steps:
   S1. Accurately weigh Sirolimus, solvent in aqueous phase and sodium dodecyl sulfate, respectively, and stir magnetically to obtain a suspension;
   S2. Perform several times of homogenization to the suspension through a microfluidic device to obtain a nanocrystals suspension;
   S3. Freeze-dry to obtain Sirolimus nanocrystals;

Preferably, the particle size d₅₀ of the nanocrystals particles is 150 to 900 nm, the microfluidization pressure is 1,000 to 2,000 bar, the homogenization is performed for 5 to 10 times, and the mass ratio of Sirolimus to sodium dodecyl sulfate is between 5:1 and 1:2.

The present invention also provides a coating solution for drug-coated balloon, the coating solution comprises an aqueous solvent, and the coating solution further comprises a plurality of core-shell structures formed by wrapping drugs in a phospholipid bilayer. The core-shell structures are dispersed in the solvent in aqueous phase. A core of the core-shell structure comprises a plurality of particles loaded with drugs. A shell of the core-shell structure is the phospholipid bilayer. The phospholipid bilayer comprises a hydrophilic group in an outer layer and a hydrophobic group in an inner layer. The plurality of particles loaded with drugs comprises a plurality of nanocrystals particles comprising drugs.

Wherein, the raw materials for preparing the nanocrystals particles include a surfactant, and the surfactant is selected from one or more of vitamin E polyethylene glycol succinate, Poloxamer 188, Poloxamer 407, Tyloxapol, docusate sodium, polyoxyl 15-hydroxystearate, polyoxyethylene (40) hydrogenated castor oil, polyoxyethylene (60) hydrogenated castor oil, polyoxyethylene (60) castor oil, polyoxyethylene (40) castor oil, polyoxyethylene (35) castor oil, polyoxyethylene (20) castor oil, polyoxyethylene (10) castor oil, Polyethylene glycol hexadecyl octadecyl ether 20, polyethylene glycol hexadecyl octadecyl ether 12, polyoxyethylene monocetyl ether (Ceteth-10), polyoxyethylene (10) lauryl ether (Brij 56), polyoxyethylene (20) lauryl ether (Brij 58), polyoxyethylene (23) lauryl ether (Brij 35), polyoxyethylene (2) lauryl ether (Brij 52), polyoxyethylene (25) oleate (Myrj 49), polyoxyethylene (40) palmitate (Myrj 52), sodium dodecyl sulfate, Tween 80, Tween 60, Tween 40, propylene glycol monolaurate, polyoxyethylene (20) lauryl ether, and polyoxyethylene monocetyl ether.
Preferably, the method for preparing nanocrystals particles comprises: anti-solvent method, high pressure homogenization method or microfluidic method;
Preferably, the anti-solvent method comprises the following steps:
   S1. Accurately weigh Sirolimus and organic solvent, respectively, and stir magnetically until a transparent and uniform phase A solution is obtained;
   S2. Accurately weigh the surfactant and the solvent in aqueous phase, respectively, and stir magnetically until a transparent and uniform phase B solution is obtained;
   S3. Add phase A solution to phase B solution quickly, and stir magnetically to obtain a nanocrystals suspension;
   S4. Add water to the suspension and mix well, and then freeze-dry to obtain the nanocrystals particles;
Preferably, the particle size d₅₀ of the nanocrystals particles is between 150 and 900 nm, and the mass ratio of Sirolimus to surfactant is between 5:1 and 1:2;
Preferably, the high pressure homogenization method comprises the following steps:
   S1. Accurately weigh Sirolimus, solvent in aqueous phase and surfactant, respectively, and stir magnetically to obtain a suspension;
   S2. Perform several times of homogenization to the suspension through a high pressure homogenizer to obtain a nanocrystals suspension;
   S3. Freeze-dry to obtain Sirolimus nanocrystals;
Preferably, the particle size d₅₀ of the nanocrystals particles is 150 to 900 nm, the homogenization pressure is 1,000 to 2,000 bar, the homogenization is performed for 5 to 10 times, and the mass ratio of Sirolimus to surfactant is between 5:1 and 1:2;
Preferably, the microfluidic method comprises the following steps:
   S1. Accurately weigh Sirolimus, solvent in aqueous phase and surfactant, respectively, and stir magnetically to obtain a suspension;
   S2. Perform several times of homogenization to the suspension through a microfluidic device to obtain a nanocrystals suspension;
   S3. Freeze-dry to obtain Sirolimus nanocrystals;

Preferably, the particle size d₅₀ of the nanocrystals particles is 150 to 900 nm, the microfluidization pressure is 1,000 to 2,000 bar, the homogenization is performed for 5 to 10 times, and the mass ratio of Sirolimus to surfactant is between 5:1 and 1:2.

The present invention has the following technical effects: nanocrystals is combined with liposome, it leverages the advantages of two types of drug carriers. The resulting phospholipid bilayer enhances the solubility of poorly soluble drugs, offers high drug loading capacity, ensures good stability, and allows for controllable drug release rates.

The present invention also provides a use of the above-mentioned drug-coated balloon in intracranial artery stenosis, wherein the dose of drug is less than or equal to 3 µg/mm², and more preferably less than or equal to 1.5 µg/mm².

The present invention also provides a use of Sirolimus-coated balloon in intracranial artery stenosis, wherein the dose of drug is less than or equal to 3 µg/mm², and more preferably less than or equal to 1.5 µg/mm².

The present invention combines nanocrystals with liposomes, and thus, the advantages of the two types of drug carriers are combined. The formed phospholipid bilayer increases the solubility of a poorly soluble drug on the drug-coated balloon, the drug loading capacity is high, the stability of the drug carriers is maintained and the crystalline form of the drug is stable, such that the drug release rate is controllable.

### Brief Description of Drawings

The features and advantages of the present invention can be further understood with reference to the drawings, and the drawings are schematic and should not be construed as any limitation on the invention. In the drawings:
FIG. 1 shows a schematic diagram of the nanocrystal-liposome composite nanocarrier in the present invention.
FIG. 2 shows the electron micrograph for the surface of Sirolimus-coated balloon in the present invention.

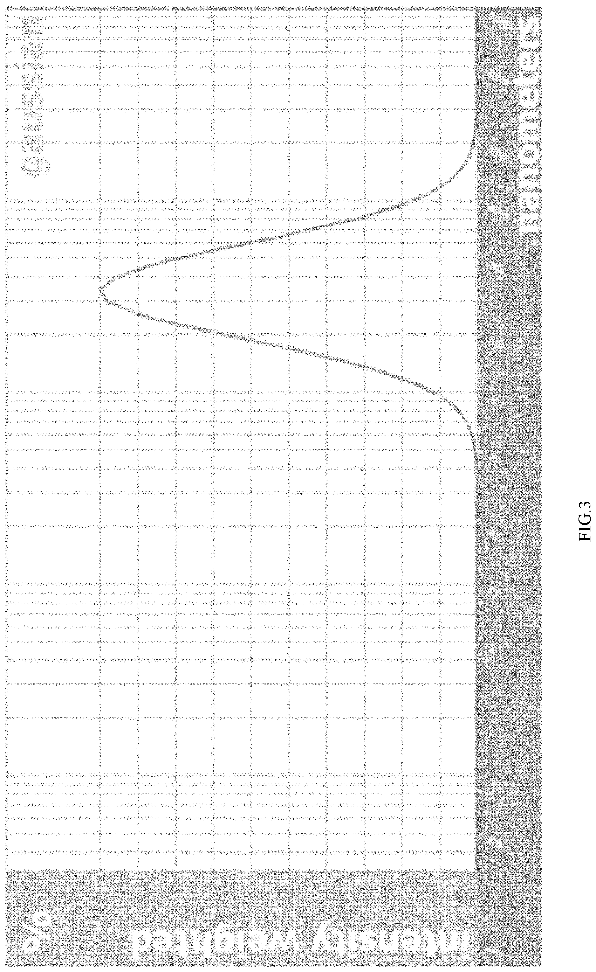

FIG. 3 shows the nanocrystals particle size distribution in the present invention.

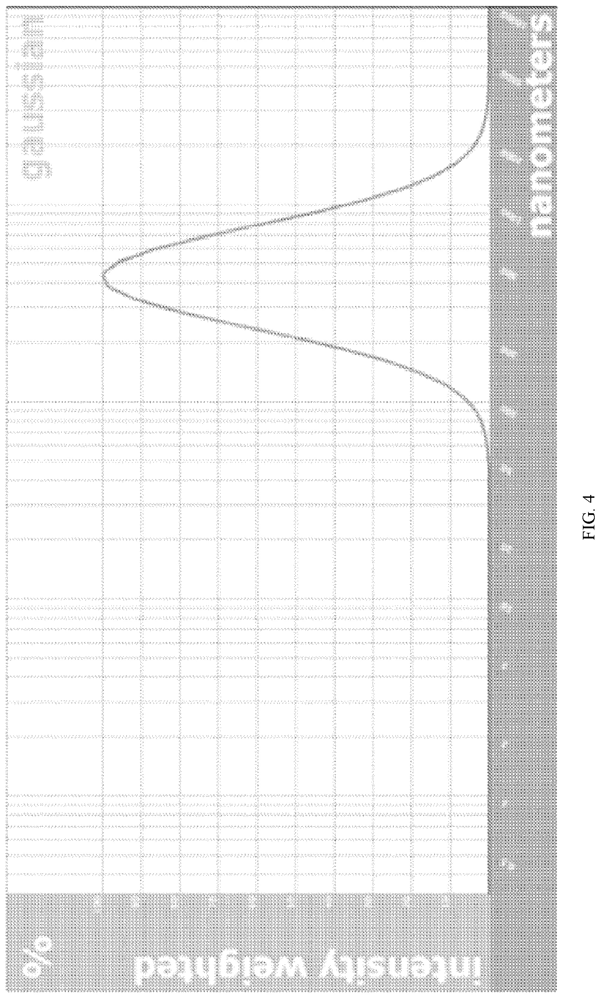

FIG. 4 shows the particle size distribution of nanocrystal-liposome composite nanocarriers in the present invention.
FIG. 5 shows crystal patterns of nanocrystal-liposome composite nanocarriers prepared by different formulations and processes before and after sterilization in the present invention (different formulae: polyoxyethylene (35) castor oil: EL35; polyoxyl 15-hydroxystearate: HS15; Tyloxapol; different processes: anti-solvent method, microfluidic method).
FIG. 6 shows the crystal patterns of nanocrystal-liposome composite nanocarriers prepared by different processes with sodium dodecyl sulfate: SDS as the surfactant before and after sterilization in the present invention and nanocrystal-liposome composite nanocarriers prepared with HS15 after sterilization.

### Embodiments

To clearly understand the objectives, technical solutions, and advantages of the embodiments of the present invention, the following detailed description is provided in conjunction with the accompanying drawings. Apparently, the examples described here are only some examples of the present invention rather than all possible examples of the present invention. Based on the examples in the present invention, all other examples obtained by those skilled in the art without expending any creative labor fall into the protection scope of the present invention.

In the examples of the present invention, a nanocrystals containing drugs is first prepared, and the drugs include Sirolimus, Zotarolimus or Everolimus. The following examples take Sirolimus as an example for description. The preparation methods of the nanocrystals include anti-solvent method, high pressure homogenization method and microfluidic method.

### Example 1A

Wherein, the anti-solvent method adopted for the Sirolimus nanocrystals comprises the following steps:
(1) Accurately weigh Sirolimus and organic solvent, respectively, and stir magnetically until a transparent and uniform phase A solution is obtained;
(2) Accurately weigh sodium dodecyl sulfate and solvent in aqueous phase, respectively, and stir magnetically until a transparent and uniform phase B solution is obtained;
(3) Add phase A solution to phase B solution quickly, and stir magnetically to obtain a nanocrystals suspension;
(4) Add water to the suspension and mix well, and then freeze-dry to obtain nanocrystals particles.

In a specific embodiment, the anti-solvent method may include the following steps, with the specific amounts of each raw material shown in the accompanying table:

**Table 1**

| Category | Raw materials and excipients | Theoretical dosage |
|---|---|---|
| Phase A | Sirolimus | 300 mg |
| | Methanol | 60 mL |
| Phase B | Sodium dodecyl sulfate | 225 mg |
| | Water | 150 g |

(1) Accurately weigh Sirolimus and methanol, respectively, and stir magnetically for 10 minutes until a transparent and uniform phase A solution is obtained;
(2) Accurately weigh sodium dodecyl sulfate and water, respectively, and stir magnetically for 10 minutes until a transparent and uniform phase B solution is obtained;
(3) Add phase A solution to phase B solution quickly, and stir magnetically for 10 minutes to obtain a nanocrystals suspension;
(4) Add 150 g of water, mix well, and freeze-dry to obtain Sirolimus nanocrystals.

Sirolimus nanocrystals particle size d₅₀ is measured, wherein d₅₀=340 nm.

### Example 1B

Wherein, the high pressure homogenization method adopted for the Sirolimus nanocrystals comprises the following steps:
(1) Accurately weigh Sirolimus, solvent in aqueous phase and sodium dodecyl sulfate, respectively, and stir magnetically to obtain a suspension;
(2) Perform several times of homogenization to the suspension through a high pressure homogenizer to obtain a nanocrystals suspension;
(3) Freeze-dry to obtain Sirolimus nanocrystals.

The specific high pressure homogenization method may specifically include the following steps, wherein specific dosage of each raw material is shown in the following table:

**Table 2**

| Raw materials and excipients | Theoretical dosage |
|---|---|
| Sirolimus | 300 mg |
| Sodium dodecyl sulfate | 225 mg |
| Water | 150 g |

(1) Accurately weigh Sirolimus, water and sodium dodecyl sulfate, respectively, and stir magnetically for 10 minutes to obtain a suspension;
(2) Perform 8 times of homogenization to the suspension through a high pressure homogenizer under a homogenization pressure of 1,300 bar to obtain a nanocrystals suspension;
(3) Freeze-dry to obtain Sirolimus nanocrystals.

Sirolimus nanocrystals particle size d₅₀ is measured, wherein d₅₀ = 512 nm.

### Example 1C

The microfluidic method adopted for the Sirolimus nanocrystals may specifically comprise the following steps, wherein specific dosage of each raw material is shown in the following table:

**Table 3**

| Raw materials and excipients | Theoretical dosage |
|---|---|
| Sirolimus | 300 mg |
| Sodium dodecyl sulfate | 225 mg |
| Water | 150 g |

(1) Accurately weigh Sirolimus, water and sodium dodecyl sulfate, respectively, and stir magnetically for 10 minutes to obtain a suspension.
(2) Perform 8 times of homogenization to the suspension through a microfluidic device, under a microfluidization pressure of 1,600 bar to obtain a nanocrystals suspension.
(3) Freeze-dry to obtain Sirolimus nanocrystals.

Sirolimus nanocrystals particle size d₅₀ is measured, wherein d₅₀ = 160 nm.

### Example 1D

Wherein, the anti-solvent method adopted for the Sirolimus nanocrystals comprises the following steps:
(1) Accurately weigh Sirolimus and organic solvent, respectively, and stir magnetically until a transparent and uniform phase A solution is obtained;
(2) Accurately weigh Tween 80 and solvent in aqueous phase, respectively, and stir magnetically until a transparent and uniform phase B solution is obtained;
(3) Add phase A solution to phase B solution quickly, and stir magnetically to obtain a nanocrystals suspension;
(4) Add water to the suspension and mix well, and then freeze-dry to obtain nanocrystals particles.

In a specific example, the anti-solvent method may specifically comprise the following steps, wherein specific dosage of each raw material is shown in the following table:

**Table 4**

| Category | Raw materials and excipients | Theoretical dosage |
|---|---|---|
| Phase A | Sirolimus | 300 mg |
| | Methanol | 60 mL |
| Phase B | Tween 80 | 150 mg |
| | Water | 150 g |

(1) Accurately weigh Sirolimus and methanol, respectively, and stir magnetically for 10 minutes to obtain a transparent and uniform phase A solution.
(2) Accurately weigh Tween 80 and water, respectively, and stir magnetically for 10 minutes to obtain a transparent and uniform phase B solution.
(3) Add phase A solution to phase B solution quickly, and stir magnetically for 10 minutes to obtain a nanocrystals suspension.
(4) Add 150 g of water, mix well, and freeze-dry to obtain Sirolimus nanocrystals.

Sirolimus nanocrystals particle size d₅₀ is measured, wherein d₅₀ = 776 nm.

### Example 1E

Wherein, the high pressure homogenization method adopted for the Sirolimus nanocrystals comprises the following steps:
(1) Accurately weigh Sirolimus, solvent in aqueous phase and Poloxamer 188, respectively, and stir magnetically to obtain a suspension;
(2) Performing several times of homogenization to the suspension through a high pressure homogenizer to obtain a nanocrystals suspension;
(3) Freeze-dry to obtain Sirolimus nanocrystals.

In a specific example, the high pressure homogenization method may specifically comprise the following steps, wherein specific dosage of each raw material is shown in the following table:

**Table 5**

| Raw materials and excipients | Theoretical dosage |
|---|---|
| Sirolimus | 300 mg |
| Poloxamer 188 | 50 mg |
| Water | 150 g |

(1) Accurately weigh Sirolimus, water and Poloxamer 188, respectively, and stir magnetically for 10 minutes to obtain a suspension.
(2) Perform 10 times of homogenization to the suspension through a high pressure homogenizer, under a microfluidization pressure of 2,000 bar to obtain a nanocrystals suspension.
(3) Freeze-dry to obtain Sirolimus nanocrystals.

Sirolimus nanocrystals particle size d₅₀ is measured, wherein d₅₀ = 225 nm.

### Example 1F

In a specific embodiment, the microfluidic method adopted for the Sirolimus nanocrystals may specifically comprise the following steps, wherein specific dosage of each raw material is shown in the following table:

**Table 6**

| Raw materials and excipients | Theoretical dosage |
|---|---|
| Sirolimus | 300 mg |
| Poloxamer 407 | 100 mg |
| Water | 150 g |

(1) Accurately weigh Sirolimus, water and Poloxamer 407, respectively, and stir magnetically for 10 minutes to obtain a suspension.
(2) Perform 2 times of homogenization to the suspension through a microfluidic device, under a microfluidization pressure of 1,800 bar to obtain a nanocrystals suspension.
(3) Freeze-dry to obtain Sirolimus nanocrystals. Sirolimus nanocrystals particle size d₅₀ is measured, wherein d₅₀ = 530 nm.

### Example 1G

In a specific example, the high-pressure nano-microfluidic method adopted for the Sirolimus nanocrystals may specifically comprise the following steps, wherein specific dosage of each raw material is shown in the following table:

**Table 7**

| Raw materials and excipients | Theoretical dosage |
|---|---|
| Sirolimus | 300 mg |
| Polyoxyethylene (35) castor oil | 20 mg |
| Water | 40 g |

(1) Accurately weigh Sirolimus, water and polyoxyethylene (35) castor oil, respectively, stir magnetically for 15 minutes with a rotation speed of 800 rpm to obtain a suspension.
(2) Perform homogenization to the suspension through a high-pressure nano-microfluidic device for 35 times under a pressure of 1,800 bar to obtain a nanocrystals suspension.
(3) Freeze-dry to obtain Sirolimus nanocrystals. Sirolimus nanocrystals particle size d₅₀ is measured, wherein d₅₀ = 520 nm.

### Example 2

Nanocrystal-liposome composite nanocarriers of core-shell structures formed by wrapping drugs in a phospholipid bilayer are prepared with the nanocrystals particles containing drugs, which have been prepared in Examples 1A to 1G (measured Sirolimus nanocrystals particle size d₅₀ = 150-900 nm). The nanocrystal-liposome composite nanocarriers can be prepared by high pressure homogenization method or microfluidic method. The high pressure homogenization method or the microfluidic method may be a two-step method or a one-step method.

Wherein, the specific dosage for each raw material is shown in the following table:

**Table 8 - Example 2A**

| Raw materials and excipients | Proportion (%) | 15 mL theoretical amount (mg) |
|---|---|---|
| Nanocrystals | 1.85 | 277.5 |
| Sodium alginate | 0.05 | 7.5 |
| Cholesterol | 0.6 | 90 |
| Lecithin | 1.2 | 180 |
| Water | 96.3 | 14.45 g |

In another specific example, specific dosage of each raw material is shown in the following table:

**Table 9 - Example 2B**

| Raw materials and excipients | Proportion (%) | 15 mL theoretical amount (mg) |
|---|---|---|
| Nanocrystals | 1.85 | 277.5 |
| Cholesterol | 0.6 | 90 |
| Lecithin or soybean phospholipid | 1.2 | 180 |
| Water | 96.35 | 14.45 g |

1. The two-step procedure for the high-pressure homogenization method may include the following steps:
   (1) Accurately weigh cholesterols, lecithin and water, respectively, stir magnetically at 60°C for 40 minutes, and then perform 5 to 10 times of homogenization through a high pressure homogenizer under a homogenization pressure of 1,000 to 2,000 bar;
   (2) Accurately weigh and add nanocrystals, stir magnetically at 60 °C for 2h, mix well, and thus obtain the nanocrystal-liposome composite nanocarriers.
   Wherein, the particle size of the nanocrystal-liposome composite nanocarriers is 200-900 nm, and the mass ratio of lecithin to cholesterols is between 40:1 and 1:2, and more preferably between 30:1 and 1:1.
2. The two-step procedure for the microfluidic method may include the following steps:
   (1) Accurately weigh cholesterols, lecithin and water, respectively, stir magnetically at 60°C for 40 minutes, and perform 5 to 10 times of homogenization through a microfluidic device under a microfluidization pressure of 1,000 to 2,000 bar;
   (2) Accurately weigh and add nanocrystals, stir magnetically at 60 °C for 2h, mix well, and thus obtain the nanocrystal-liposome composite nanocarriers.
   Wherein, the particle size of the nanocrystal-liposome composite nanocarriers is 200 to 900 nm, and the mass ratio of lecithin to cholesterols is 40:1 to 1:2, and more preferably 30:1 to 1:1.
3. The one-step procedure for the high pressure homogenization method may include the following steps:
   (1) Accurately weigh cholesterols and lecithin, respectively, dissolve them uniformly at 60 °C to obtain an oil phase;
   (2) Accurately weigh freeze-dried powder of nanocrystals and water, respectively, and mix well at 60 °C by magnetic stirring to obtain a water phase;
   (3) Add the water phase to the oil phase and mix at 60 °C for 20 minutes to obtain a primary emulsion;
   (4) Perform 5 to 10 times of homogenization under a homogenization pressure of 1,000 to 2,000 bar, and then freeze-dry to obtain the nanocrystal-liposome composite nanocarriers.
   Wherein, the particle size of nanocrystal-liposome composite nanocarriers is 200 to 900 nm, and the mass ratio of lecithin or soybean phospholipid to cholesterols is 40:1 to 1:2, and more preferably 30:1 to 1:1.
4. The one-step procedure for the microfluidic method may include the following steps:
   (1) Accurately weigh cholesterols and lecithin, respectively, dissolve them uniformly at 60 °C to obtain an oil phase;
   (2) Accurately weigh the freeze-dried powder of nanocrystals and water, respectively, and mix well at 60 °C by magnetic stirring to obtain a water phase;
   (3) Add the water phase to the oil phase and mix at 60 °C for 20 minutes to obtain a primary emulsion;
   (4) Perform 5 to 10 times of homogenization to the primary emulsion through a microfluidic device under a homogenization pressure of 1,000 to 2,000 bar, and then freeze-dry to obtain the nanocrystal-liposome composite nanocarriers.

Wherein, the particle size of the nanocrystal-liposome composite nanocarriers is between 200 and 900 nm, and the mass ratio of lecithin to cholesterols is between 40:1 and 1:2, and more preferably between 30:1 and 1:1.

The phospholipids in the present application can be replaced with one or more of yolk phospholipid, soybean phospholipid, sunflower phospholipid, hydrogenated soybean phospholipid, hydrogenated yolk phospholipid, 1,2-diformyl-sn-glycero-3-phosphocholine, 1,2-dipalmitoyl-sn-glycero-3-phosphocholine, 1,2-distearoyl-sn-glycero-3-phosphocholine, 1,2-dioleoyl-sn-glycero-3-phosphocholine, 1,2-dierucoyl-sn-glycero-3-phosphocholine, 1-dipalmitoyl-dioleoyl-sn-glycero-3-phosphocholine, 1,2-diformyl-sn-glycero-3-phospho-rac-glycerol sodium salt, 1,2-dipalmitoyl-sn-glycero-3-phospho-rac-glycerol sodium salt, 1,2-distearoyl-sn-glycero-3-phospho-rac-glycerol sodium salt, 1,2-dioleoyl-sn-glycero-3-phospho-rac-glycerol sodium salt, 1-dipalmitoyl-dioleoyl-sn-glycero-3-phospho-rac-glycerol sodium salt, 1,2-diformyl-sn-glycero-3-phosphoethanolamine, 1,2-dipalmitoyl-sn-glycero-3-phosphoethanolamine, 1,2-distearoyl-sn-glycero-3-phosphoethanolamine, 1,2-dioleoyl-sn-glycero-3-phosphoethanolamine, 1,2-dioleoyl-sn-glycero-3-phospho-L-serine sodium salt, 1,2-dipalmitoyl-sn-glycero-3-phosphate monosodium salt, N-(carbonyl-methoxypolyethylene glycol-5000)-1,2-dipalmitoyl-sn-glycero-3-phosphoethanolamine sodium salt, N-(carbonyl-methoxypolyethylene glycol-2000)-1,2-distearoyl-sn-glycero-4-phosphoethanolamine sodium salt, and 1,2-dioleoyl-3-trimethylpropanyl ammonium chloride.

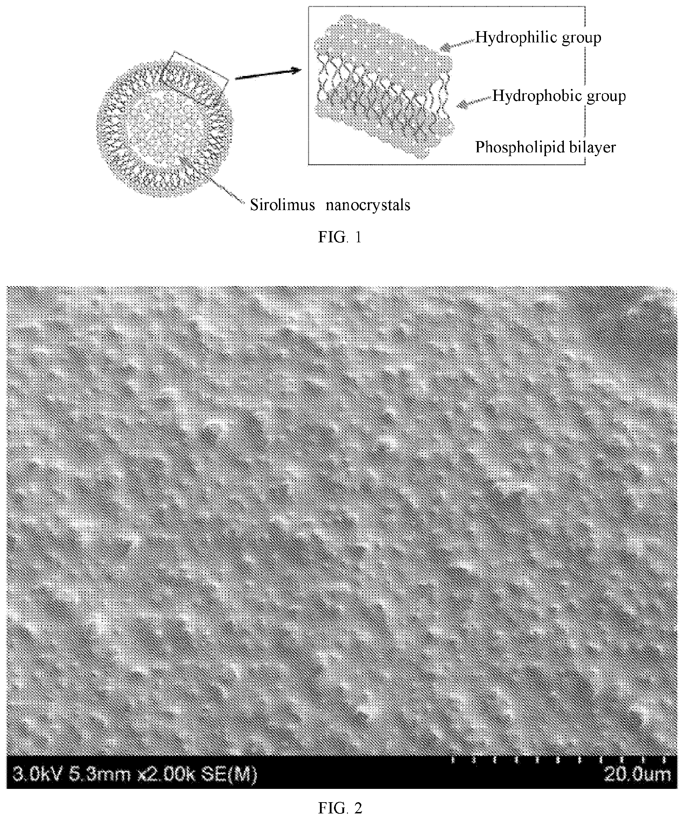

The nanocrystal-liposome composite nanocarriers prepared by the above-mentioned preparation method in the present invention are of core-shell structures. A core of the core-shell structure comprises a plurality of particles loaded with drugs. A shell of the core-shell structure is the phospholipid bilayer. The phospholipid bilayer comprises a hydrophilic group in an outer layer and a hydrophobic group in an inner layer. Specific core-shell structures as described are as shown in FIG. 1.

The freeze-dried powder of nanocrystal-liposome composite nanocarriers is redissolved in pure water to form a coating solution, which undergoes high-frequency ultrasonic atomization to generate a fog, and the fog may form an aerosol with air. Under a stable pressure, the aerosol is pushed out from the nozzle by nitrogen. A fixture is utilized to connect the support module, the balloon moves radially and axially at a constant speed in the air flow, and the atomized particles carried in the air flow, after contacting with the surface of the balloon, deposit on the surface of the balloon, forming a liquid film, which will be dried to obtain the coating structure required for the drug-coated balloon.

In the present invention, nanocrystals and liposomes are combined, and thus, the advantages of the two drug carriers are combined. The formed phospholipid bilayer increases the solubility of a poorly soluble drug, the drug loading capacity is high, the stability is good, and the drug release rate is controllable.

In particular, it should be noted that an aqueous solvent is used to prepare the coating solution for drug-coated balloon in the present invention. The coating solution for drug-coated balloon based on aqueous phase has technical effects as follows: 1. the firmness is good; 2. there is little residue of organic solvent; 3. it could be applied to both aqueous-soluble and non-aqueous-soluble drugs.

### Comparative Example 1

The preparation method is identical to that of the nanocrystal-liposome composite nanocarriers except that cholesterols are not added. Consequently, a nanocrystal-phospholipid composite is obtained, wherein the nanocrystals are encapsulated by monolayer liposomes in the nanocrystal-phospholipid composite. The results of Example 1 and Comparative Example 1 are compared as follows:

**Table 10**

| Sample | Group | Shell structure | Particle size d₅₀ (nm) |
|---|---|---|---|
| Comparative Example 1 | Nanocrystal-phospholipid composite | Monolayer | 372 |
| Example 1A - Example 2B | Nanocrystal-liposome composite nanocarriers | Bilayer | 421 |
| Example 1B - Example 2B | Nanocrystal-liposome composite nanocarriers | Bilayer | 553 |
| Example 1C - Example 2B | Nanocrystal-liposome composite nanocarriers | Bilayer | 226 |
| Example 1D - Example 2B | Nanocrystal-liposome composite nanocarriers | Bilayer | 872 |
| Example 1E - Example 2B | Nnanocrystal-liposome composite nanocarriers | Bilayer | 389 |
| Example 1F - Example 2B | Nanocrystal-liposome composite nanocarriers | Bilayer | 783 |
| Example 1G - Example 2B | Nanocrystal-liposome composite nanocarriers | Bilayer | 427 |

### Test example - dose verification for intracranial drug-coated balloon

The purpose of this test (MTT(3-(4,5-dimethylthiazol-2)-2,5-diphenyltetrazolium bromide)) is to evaluate the cytotoxicity of Sirolimus coating with different drug concentrations and coating formulae on L-929 cells, C6 cells, and HT22 cells quantitatively.

For both test sample and positive control, the required dose is calculated based on different groups. They are dissolved in pure DMSO, and then a serum-containing medium (10 ml per group) is added for adjusting the concentration of DMSO to 0.1%. The serum-containing medium is added to each group of solution based on volume ratio, and thus, three subgroups, i.e., 100%, 50%, and 25% subgroups, are obtained.

Negative control solution: the serum-containing medium is added to 0.01 ml of pure DMSO, so as to adjust the DMSO concentration to 0.1%. Thus, 10 ml of negative control solution is obtained. Medium control solution: take 10 ml of serum-containing medium as the medium control solution.

Inoculate L-929, C6, and HT22 cells into 96-well plates and culture for 24 hours, and after a subconfluent cell layer is formed, expose them to the corresponding test sample, control, and medium control solution. After 24 hours of cell incubation, remove the supernatant, and add MTT solution to each well. After 2 hours of cell culture plate incubation, remove the MTT solution, and add 100 uL of isopropanol. Place the culture plate into the ELIASA, read the absorbance value under 570 nm wavelength with 650 nm as the reference wavelength. The cytotoxicity is detected by measuring cell viability, which is determined by the percentage of OD570 (mean OD570) relative to the medium control in each dosing group.

In Test 1, the deviations of mean OD570 of L929 and C6 cells in the left side (column 2) and the right side (column 11) microplates relative to mean values of both sides are 2.61% and 4.14% respectively, which are less than 15%. The cell viabilities of the negative control are 101.51% and 95.30% respectively.

In Test 2, the deviations of mean OD570 of L929 cells in the left side (column 2) and the right side (column 11) microplates based on Formulae A, B, and C relative to mean values of both sides are 8.59%, 5.06%, and 10.27% respectively, and the deviations of mean OD570 of C6 cells in the left side (column 2) and the right side (column 11) microplates based on Formulae A, B, and C relative to mean values of both sides are 3.54%, 6.13%, and 5.65% respectively, all of which are less than 15%. In the negative control group, the cell viabilities of L929 cells based on Formulae A, B, and C are 98.45%, 102.47% and 97.37% respectively, and the cell viabilities of HT22 cells based on Formulae A, B, and C are 100.44%, 98.96% and 101.52% respectively. Based on the above results, the test is valid.

The cell viabilities of L929 cells at 25% in TA1 and TA2 groups are 77.64% and 64.08% respectively, and the cell viabilities of C6 cells at 25% are 79.33% and 75.71% respectively. This indicates that the drug concentration of the coating should be within 1 to 2 µg/mm² for achieving an inhibitory effect on fibroblasts without influence on nerve cells.

Perform the second screening to the two cell lines: L929 and HT22 at concentrations of 1.0, 1.5 and 2.0 µg/mm², and three formulae of drugs for the coating (A: Sirolimus + lac ammonium salt; B: nanocrystals + lecithin + cholesterol (Example 2); C: PLGA+ lecithin+ cholesterol) are used to perform toxicity test on the cells.

The results show that the three formulae have an inhibitory effect on L929 cells at 1.0 to 2 µg/mm², but the Formulae A, B and C have no inhibitory effect on HT 22 cells at 1.0 and 1.5 µg/mm², the three formulae have an inhibitory effect on HT 22 cells at 2.0 µg/mm². Therefore, when the concentrations of Formulae A, B and C are less than or equal to 1.5 µg/mm², Formulae A, B and C can achieve an inhibitory effect on fibroblasts without influence on nerve cells.

Therefore, it can be determined that the inhibitory dose of Sirolimus-coated balloon in intracranial vascular restenosis is less than or equal to 1.5 µg/mm².

### Animal test

### 1. Grouping test animals

A vascular implantation experiment is conducted on New Zealand rabbits:
In the test group, 24 animals are implanted, and the implanted sites are bilateral iliac arteries. For each group, 6 test products are placed (Example 2B prepared by nanocrystals from Examples 1A-F is used), 12 animals are dissected immediately after the operation, and 12 animals are dissected 7 days (7d) after the operation.

In control group 1 (Comparative Example 1), 4 animals are implanted, and the implanted sites are bilateral iliac arteries. For each group, 1 control balloon catheter is placed, 2 animals are dissected immediately after the operation, and 2 animals are dissected 7 days after the operation.

In control group 2 (Comparative Example 2 - commercially available Magic touch Sirolimus-coated balloon), 4 animals are implanted, and the implanted sites are bilateral iliac arteries. 2 animals are dissected immediately after the operation, and 2 animals are dissected 7 days after the operation.

### 2. Operating procedure

Heparin sodium injection is administrated intravenously before the operation, and the coagulation time should be monitored to ensure that the ACT is greater than 500 during the operation. During the operation, real-time monitoring of ECG should be performed, and some indicators like blood pressure and heart rate should be observed. The drug-coated balloon dilatation is performed in accordance with the standard operating procedures of the laboratory. The drug-coated balloon is guided to the blood vessel through the carotid artery with a guide catheter and a 0.014 guide wire, and, after reaching the target location of iliac artery, the drug-coated balloon is expanded with a pressure pump under appropriate pressure so that the drugs are attached to the blood vessel wall. Record the distance of the balloon from the site of vascular bifurcation. Hold the pressure for 60 s, and then deflate the balloon, and withdraw the balloon delivery system from the body. At the end of the operation, withdraw all devices and instruments from the bodies of test animals.

Immediately after the operation, dissect test group (12 animals) in situ, control group 1 (2 animals) and control group 2 (2 animals), incise the skin to expose the target blood vessel, and cut out the target vascular tissue at 2 to 3 mm upstream and downstream from the operation site. After anesthesia resuscitation, transfer the test animals to the ICU for monitoring.

### 3. Tissue sample extraction

After the rabbit blood vessel samples are weighed, cut them into pieces, and homogenize them according to the ratio of tissue to 50% methanol of 1:4 (w:v). The homogenate should be partially frozen and stored below -20 °C. Accurately transfer 20 µL of homogenate into a 1.5 mL EP tube with a pipette, add 180 µL of blank matrix, and mix well for use. Take 100 µL of standard curve sample and quality control sample, add the precipitant according to the ratio of sample to precipitant of 1:3 (v:v), mix well, centrifuge at 12,000 rpm for 10 minutes at 4°C, and transfer an appropriate amount of supernatant into a new 96-well plate for use.

In Example 2B, Comparative Example 1 and Comparative Example 2, the content in each balloon is about 1.5 µg/mm². The nanocrystal-phospholipid composite is monolayer, while the nanocrystal-liposome composite nanocarrier is a bilayer. The test results are shown in the following table:

**Table 11**

| Group | Survival for test (Days) | Sample size | Mean drug content per unit tissue (ng/mg) |
|---|---|---|---|
| Comparative Example 1 - nanocrystal-phospholipid composite | 0 | 2 (bilateral) | 68.2 ±8.9 |
| | 7 | 2 (bilateral) | 3.5±2.6 |
| Comparative Example 2 - commercially available Magic touch Sirolimus-coated balloon | 0 | 2 (bilateral) | 80.6±48.3 |
| | 7 | 2 (bilateral) | 12.7±8.5 |
| Example 1A - Example 2B | 0 | 2 (bilateral) | 312.6±62.5 |
| | 7 | 2 (bilateral) | 43.2±11.6 |
| Example 1B - Example 2B | 0 | 2 (bilateral) | 190.1±47.5 |
| | 7 | 2 (bilateral) | 22.7±4.7 |
| | | | |
| Example 1C - Example 2A | 0 | 2 (bilateral) | 203.2±65.0 |
| | 7 | 2 (bilateral) | 26.1±8.4 |
| Example 1D - Example 2A | 0 | 2 (bilateral) | 179.3±51.4 |
| | 7 | 2 (bilateral) | 37.3±10.7 |
| Example 1E - Example 2A | 0 | 2 (bilateral) | 250.6±66.6 |
| | 7 | 2 (bilateral) | 28.5±9.7 |
| Example 1F - Example 2A | 0 | 2 (bilateral) | 222.6±69.9 |
| | 7 | 2 (bilateral) | 26.1+10.3 |
| Example 1G - Example 2A | 0 | 2 (bilateral) | 237.1±105.4 |
| | 7 | 2 (bilateral) | 12.5±4.4 |

It can be seen from the above table that the content of nanocrystal-liposome composite nanocarrier (Example 2) in the tissue at 7 days is significantly higher than that of Comparative Example 1 (nanocrystal-phospholipid composite) and Comparative Example 2. The average drug content of nanocrystal-liposome composite nanocarrier per unit tissue at 7 days is increased by more than 2 times relative to the Comparative Examples, and so the drug sustained release effect of the nanocrystal-liposome composite nanocarrier of the present invention is better.

### Test Example - verification test of particle size and crystal form of the present invention

The present invention also systematically compares the parameters of nanocrystals and liposomes under different formulas and processes, and verifies that their crystal forms are substantially stable. Specific test conditions and results are provided in the following table and text:

### Nanocrystals preparation process:

### Anti-solvent method:

(1) Accurately weigh Sirolimus and methanol, respectively, and stir magnetically until a transparent and uniform phase A solution is obtained;
(2) Accurately weigh the surfactant and the solvent in aqueous phase, respectively, and stir magnetically until a transparent and uniform phase B solution is obtained;
(3) Add phase A solution to phase B solution quickly, and stir magnetically to obtain a nanocrystals suspension;

### Microfluidic method:

(1) Accurately weigh Sirolimus, surfactant and water, respectively, and stir magnetically for 5 to 15 minutes to obtain a suspension.
(2) Perform 30 to 40 times of homogenization to the suspension through a microfluidic device under a microfluidization pressure of 1,600-2,000 bar to obtain a nanocrystals suspension.

### Liposome preparation process:

(1) Accurately weigh cholesterols, phospholipid and water, respectively, dissolve them uniformly at 50 to 80 °C and the nanocrystals suspension is added;
(2) Perform 15 to 25 times of homogenization through a microfluidic device, under a microfluidization pressure of 1,200-1,500 bar to obtain the nanocrystal-liposome composite nanocarriers.

**Table 12 Process optimization for various parameters of nanocrystals and liposomes**

| Kinds | Raw materials and excipients | Sodium dodecyl sulfate: SDS; Polyoxyethylene (35) castor oil: EL35; polyoxyl 15-hydroxystearate: HS15 | | | | | | |
|---|---|---|---|---|---|---|---|---|
| | | Anti-solvent method | | | | Microfluidic method | | |
| | | Formula 0 (SDS) | Formula 1 (EL35) | Formula 2 (HS15) | Formula 3 (Teloxabol) | Formula 4 (EL35) | Formula 5 (HS15) | Formula 6 (Teloxapore) |
| Nanocrystals | Sirolimus (mg) | 300 | 300 | 300 | 300 | 300 | 300 | 300 |
| | Methanol (ml) | 10 | 10 | 10 | 10 | / | / | / |
| | Surfactant (mg) | 200 | 200 | 200 | 200 | 200 | 200 | 200 |
| | Water (g) | 28.96 | 28.96 | 28.96 | 28.96 | 28.96 | 28.96 | 28.96 |
| Liposome | Phospholipid (mg) | 360 | 360 | 360 | 360 | 360 | 360 | 360 |
| | Cholesterol (mg) | 180 | 180 | 180 | 180 | 180 | 180 | 180 |
| Total (g) | | 30 | 30 | 30 | 30 | 30 | 30 | 30 |
| Nanocrystals | d₅₀(nm) | 518.22 | 124.43 | 956.79 | 699.45 | 296.2 | 224.4 | 113.5 |
| Liposome | d₅₀(nm) | 209.08 | 107.18 | 1181.60 | 231.01 | 145.9 | 256.8 | 96.17 |
| Whether it is isomorphic with the active pharmaceutical ingredient (API) or not | Before sterilization | Most of them are amorphous | Yes | Yes | Yes | Yes | Yes | Yes |
| | After sterilization | Most of them are mixed crystals | Yes | Yes | Yes | Yes | Yes | Yes |

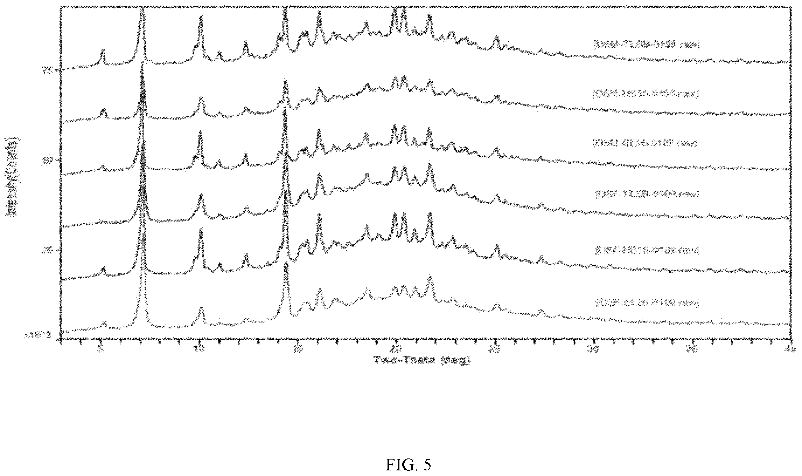

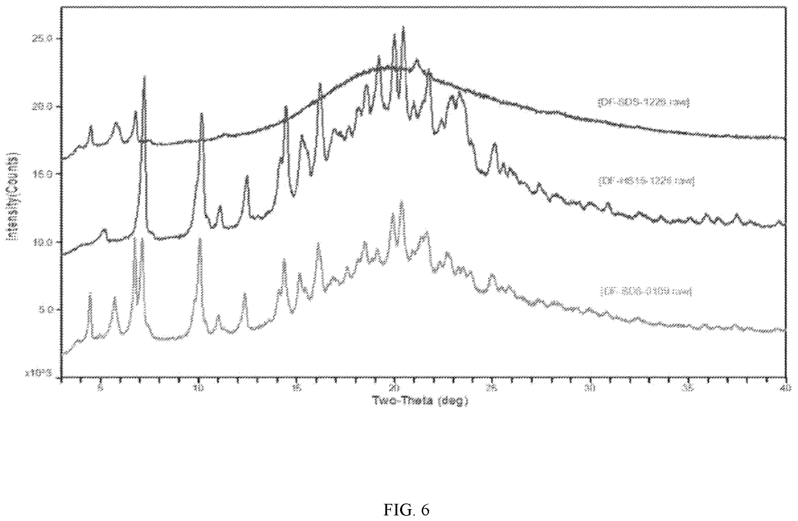

As shown in the table above, the crystal patterns of Sirolimus nanocrystal-liposome composite nanocarriers prepared by different formulae and different processes are shown in FIGS. 5 and 6. FIG. 5 shows Formula 6, Formula 5, Formula 4, Formula 3, Formula 2 and Formula 1 after sterilization respectively from top to bottom. FIG. 6 shows Formula 0 before sterilization, Formula 2 after sterilization, and Formula 0 after sterilization respectively from top to bottom.

From Table 12, it can be seen that for SDS, EL35 and Teloxabol, particle size less than 1,000 nm, which is in line with the range of typical nano preparations, of the carriers can be prepared by anti-solvent method and microfluidic method. For HS15, the particle size of the prepared carriers is slightly greater than 1,000 nm, but the particle size of carriers prepared by microfluidic method is below 1,000 nm, which is in line with the range of typical nano preparations. For HS15, EL35 and Teloxabol, the carriers prepared by anti-solvent method and microfluidic method have a crystal form as same as that of the API before and after sterilization, that is, the crystal form remains stable. For SDS, the carriers prepared by the anti-solvent method are amorphous before sterilization, but become mixed crystals after sterilization. Therefore, EL35 and Teloxapol are most suitable for the preparation of Sirolimus nanocrystal-liposome nanocarriers.

The above test examples are only used to demonstrate the technical feasibility of the preparation process, and do not represent any limitation on the range. In the above examples, products with similar quality indicators may be obtained based on a ratio of Sirolimus to surfactant within 1.5:1 to 300:1. The information on formulae (e.g., the ratio of Sirolimus to phospholipid and the ratio of phospholipid to cholesterol) can also be changed to prepare products with similar quality indicators.

The present invention has also systematically verified the stability of crystal form for the nanocrystal-liposome composite nanocarriers, and confirmed the stability of crystal form, thus providing a guarantee for the sustained release of Sirolimus. As shown in the table below:

**Table 13**

| Condition | Time (m) | Content | Crystal form |
|---|---|---|---|
| Long-term | 3 | Stable | Unchanged |
| Accelerated | 3 | Stable | Unchanged |

In accordance with the "Guidelines for the Stability of APIs and Preparations" in the Chinese Pharmacopoeia (2020 Edition), the nanocrystal-liposome composite nanocarriers are placed under accelerated conditions (temperature 40 °C±2 °C, relative humidity 75%±5%) and long-term conditions (temperature 25 °C±2 °C, relative humidity 60%±5%) respectively after commercial packaging of balloons, and the corresponding content and crystal form are investigated at 0 and 3 months. The test results show that the nanocrystal-liposome composite nanocarriers remain stable for 3 months under accelerated conditions and long-term conditions.

## Claims

1. A coating solution for drug-coated balloon, **characterized in that** the coating solution comprises an aqueous solvent, and the coating solution further comprises a plurality of core-shell structures formed by wrapping drugs in a phospholipid bilayer, and the core-shell structures are dispersed in the solvent in aqueous phase, wherein, a core of the core-shell structure comprises a plurality of particles loaded with drugs, and a shell of the core-shell structure is a phospholipid bilayer comprising a hydrophilic group in the outer layer and a hydrophobic group in the inner layer, the plurality of particles loaded with drugs comprises a plurality of nanocrystals particles comprising drugs.

2. The coating solution according to claim 1, **characterized in that** the particle size of the core-shell structures is 200 to 900 nm;
preferably, the particle size d₅₀ of the nanocrystals particles is 150 to 900 nm; wherein in the preparation process of nanocrystals particles, a surfactant is used;
preferably, the surfactant is selected from one or more of vitamin E polyethylene glycol succinate, Poloxamer 188, Poloxamer 407, Tyloxapol, docusate sodium, polyoxyl 15-hydroxystearate, polyoxyethylene (40) hydrogenated castor oil, polyoxyethylene (60) hydrogenated castor oil, polyoxyethylene (60) castor oil, polyoxyethylene (40) castor oil, polyoxyethylene (35) castor oil, polyoxyethylene (20) castor oil, polyoxyethylene (10) castor oil, polyethylene glycol hexadecyl octadecyl ether 20, polyethylene glycol hexadecyl octadecyl ether 12, polyoxyethylene monocetyl ether (Ceteth-10), polyoxyethylene (10) lauryl ether (Brij 56), polyoxyethylene (20) lauryl ether (Brij 58), polyoxyethylene (23) lauryl ether (Brij 35), polyoxyethylene (2) lauryl ether (Brij 52), polyoxyethylene (25) oleate (Myrj 49), polyoxyethylene (40) palmitate (Myrj 52), sodium dodecyl sulfate, Tween 80, Tween 60, Tween 40, propylene glycol monolaurate, polyoxyethylene (20) lauryl ether, and polyoxyethylene monocetyl ether;
preferably, the drugs include Sirolimus, Zotarolimus and Everolimus, Tacrolimus, Temsirolimus, Pimecrolimus, Deforolimus, and Ridaforolimus;
preferably, the raw materials for preparing the phospholipid bilayer include phospholipids and cholesterols;
preferably, the phospholipids include one or more of yolk lecithin, soybean phospholipid, hydrogenated yolk lecithin, hydrogenated soybean phospholipid, cephalin, phosphatidylethanolamine, dimyristyl phosphatidylcholine (DMPC), stearamide (SA), sunflower phospholipid, 1,2-diformyl-sn-glycero-3-phosphocholine, 1,2-dipalmitoyl-sn-glycero-3-phosphocholine, 1,2-distearoyl-sn-glycero-3-phosphocholine, 1,2-dioleoyl-sn-glycero-3-phosphocholine, 1,2-dierucoyl-sn-glycero-3-phosphocholine, 1-dipalmitoyl-dioleoyl-sn-glycero-3-phosphocholine, 1,2-diformyl-sn-glycero-3-phospho-rac-glycerol sodium salt, 1,2-dipalmitoyl-sn-glycero-3-phospho-rac-glycerol sodium salt, 1,2-distearoyl-sn-glycero-3-phospho-rac-glycerol sodium salt, 1,2-dioleoyl-sn-glycero-3-phospho-rac-glycerol sodium salt, 1-dipalmitoyl-dioleoyl-sn-glycero-3-phospho-rac-glycerol sodium salt, 1,2-diformyl-sn-glycero-3-phosphoethanolamine, 1,2-dipalmitoyl-sn-glycero-3-phosphoethanolamine, 1,2-distearoyl-sn-glycero-3-phosphoethanolamine, 1,2-dioleoyl-sn-glycero-3-phosphoethanolamine, 1,2-dioleoyl-sn-glycero-3-phospho-L-serine sodium salt, 1,2-dipalmitoyl-sn-glycero-3-phosphate monosodium salt, N-(carbonyl-methoxypolyethylene glycol-5000)-1,2- dipalmitoyl -sn-glycero-3-phosphoethanolamine sodium salt, N-(carbonyl-methoxypolyethylene glycol-2000)-1,2-distearoyl-sn-glycero-4-phosphoethanolamine sodium salt, and 1,2-dioleoyl-3-trimethylpropanyl ammonium chloride;
the cholesterols include cholesterol and DC cholesterol;
preferably, the mass ratio of the phospholipid to cholesterols is 40:1 to 1:2, and more preferably 30:1 to 1:1.

3. A coating material for drug-coated balloon, **characterized in that** the material comprises a plurality of core-shell structures formed by wrapping drugs in a phospholipid bilayer, wherein a core of the core-shell structures comprises a plurality of particles loaded with drugs, and a shell of the core-shell structures is the phospholipid bilayer comprising a hydrophilic group in the outer layer and a hydrophobic group in the inner layer, and the plurality of particles loaded with drugs comprise a plurality of nanocrystals particles comprising drugs.

4. The material according to claim 3, **characterized in that** the particle size of the core-shell structures is 200 to 900 nm;
preferably, the particle size d₅₀ of the nanocrystals particles is 150 to 900 nm; in the preparation process of nanocrystals particles, a surfactant is used;
preferably, the surfactant is selected from one or more of vitamin E polyethylene glycol succinate, Poloxamer 188, Poloxamer 407, Tyloxapol, docusate sodium, polyoxyl 15-hydroxystearate, polyoxyethylene (40) hydrogenated castor oil, polyoxyethylene (60) hydrogenated castor oil, polyoxyethylene (60) castor oil, polyoxyethylene (40) castor oil, polyoxyethylene (35) castor oil, polyoxyethylene (20) castor oil, polyoxyethylene (10) castor oil, Polyethylene glycol hexadecyl octadecyl ether 20, polyethylene glycol hexadecyl octadecyl ether 12, polyoxyethylene monocetyl ether (Ceteth-10), polyoxyethylene (10) lauryl ether (Brij 56), polyoxyethylene (20) lauryl ether (Brij 58), polyoxyethylene (23) lauryl ether (Brij 35), polyoxyethylene (2) lauryl ether (Brij 52), polyoxyethylene (25) oleate (Myrj 49), polyoxyethylene (40) palmitate (Myrj 52), sodium dodecyl sulfate, Tween 80, Tween 60, Tween 40, propylene glycol monolaurate, polyoxyethylene (20) lauryl ether, and polyoxyethylene monocetyl ether;
preferably, the drugs include Sirolimus, Zotarolimus and Everolimus, Tacrolimus, Temsirolimus, Pimecrolimus, Deforolimus, and Ridaforolimus;
preferably, the raw materials for preparing the phospholipid bilayer comprise phospholipid and cholesterols; preferably, the phospholipids include one or more of yolk lecithin, soybean phospholipid, hydrogenated yolk lecithin, hydrogenated soybean phospholipid, cephalin, phosphatidylethanolamine, dimyristyl phosphatidylcholine (DMPC), stearamide (SA), sunflower phospholipid, 1,2-diformyl-sn-glycero-3-phosphocholine, 1,2-dipalmitoyl-sn-glycero-3-phosphocholine, 1,2-distearoyl-sn-glycero-3-phosphocholine, 1,2-dioleoyl-sn-glycero-3-phosphocholine, 1,2-dierucoyl-sn-glycero-3-phosphocholine, 1-dipalmitoyl-dioleoyl-sn-glycero-3-phosphocholine, 1,2-diformyl-sn-glycero-3-phospho-rac-glycerol sodium salt, 1,2-dipalmitoyl-sn-glycero-3-phospho-rac-glycerol sodium salt, 1,2-distearoyl-sn-glycero-3-phospho-rac-glycerol sodium salt, 1,2-dioleoyl-sn-glycero-3-phospho-rac-glycerol sodium salt, 1-dipalmitoyl-dioleoyl-sn-glycero-3-phospho-rac-glycerol sodium salt, 1,2-diformyl-sn-glycero-3-phosphoethanolamine, 1,2-dipalmitoyl-sn-glycero-3-phosphoethanolamine, 1,2-distearoyl-sn-glycero-3-phosphoethanolamine, 1,2-dioleoyl-sn-glycero-3-phosphoethanolamine, 1,2-dioleoyl-sn-glycero-3-phospho-L-serine sodium salt, 1,2-dipalmitoyl-sn-glycero-3-phosphate monosodium salt, N-(carbonyl-methoxypolyethylene glycol-5000)-1,2- dipalmitoyl -sn-glycero-3-phosphoethanolamine sodium salt, N-(carbonyl-methoxypolyethylene glycol-2000)-1,2-distearoyl-sn-glycero-4-phosphoethanolamine sodium salt, and 1,2-dioleoyl-3-trimethylpropanyl ammonium chloride;
the cholesterols include cholesterol and DC cholesterol;
preferably, the mass ratio of phospholipid to cholesterols is 40:1 to 1:2, and more preferably 30:1 to 1:1.

5. A drug-coated balloon, **characterized in that** the surface of the drug-coated balloon is coated with the coating solution of any of claims 1-2 or the material of any of claims 3-4.

6. A use of the drug-coated balloon of claim 5 in the treatment of atherosclerosis, stenosis, and/or restenosis of coronary arteries, peripheral vessels, or intracranial artery vessels.

7. A preparation method of a coating solution for drug-coated balloon, **characterized in that** the method comprises the following steps:
(1) preparing a plurality of nanocrystals particles loaded with drugs; the method for preparing the nanocrystals particles comprises: anti-solvent method, high pressure homogenization method or microfluidic method;
(2) accurately weigh cholesterols, phospholipids and solvents in aqueous phase, respectively, and obtain a milky white solution after magnetic stirring;
(3) add nanocrystals particles to the milky white solution, mix well by magnetic stirring to obtain the coating solution for drug-coated balloon;
wherein, the coating solution comprises an aqueous solvent, and the coating solution further comprises a plurality of core-shell structures formed by wrapping drugs in a phospholipid bilayer, the core-shell structures are dispersed in the solvent in aqueous phase, wherein, a core of the core-shell structures comprises a plurality of particles loaded with drugs, and a shell of the core-shell structures is a phospholipid bilayer comprising a hydrophilic group in the outer layer and a hydrophobic group in the inner layer.

8. A method according to claim 7, **characterized in that** after Step (3), there is Step (4) for adding a thickener to the above-mentioned solution, and mixing well by magnetic stirring to obtain the coating solution for drug-coated balloon;
preferably, the thickener comprises sodium alginate;
preferably, in Step (2), after magnetic stirring, 5 to 10 times of homogenization are performed by a high pressure homogenizer or a microfluidic device to obtain a milky white solution;
preferably, the drugs in Step S1 include Sirolimus, and the organic solvents include methanol; preferably, the mass ratio of Sirolimus to sodium dodecyl sulfate is 5:1 to 1:2;
preferably, the anti-solvent method comprises the following steps:
S1. Accurately weigh Sirolimus and organic solvent, respectively, and stir magnetically until a transparent and uniform phase A solution is obtained;
S2. Accurately weigh sodium dodecyl sulfate and solvent in aqueous phase, respectively, and stir magnetically until a transparent and uniform phase B solution is obtained;
S3. Add phase A solution to phase B solution quickly, and stir magnetically to obtain a nanocrystals suspension;
S4. Add water to the suspension and mix well, and then freeze-dry to obtain nanocrystals particles;
preferably, the particle size d₅₀ of the nanocrystals particles is 150 to 900 nm, and the mass ratio of Sirolimus to sodium dodecyl sulfate is 5:1 to 1:2;
preferably, the high pressure homogenization method comprises the following steps:
S1. Accurately weigh Sirolimus, solvent in aqueous phase and sodium dodecyl sulfate, respectively, and stir magnetically to obtain a suspension;
S2. Perform several times of homogenization to the suspension through a high pressure homogenizer to obtain a nanocrystals suspension;
S3. Freeze-dry to obtain Sirolimus nanocrystals;
preferably, the particle size d₅₀ of the nanocrystals particles is between 150 and 900 nm, the homogenization pressure is 1,000 to 2,000 bar, the homogenization is performed for 5 to 10 times, and the mass ratio of Sirolimus to sodium dodecyl sulfate is between 5:1 and 1:2;
preferably, the microfluidic method comprises the following steps:
S1. Accurately weigh Sirolimus, solvent in aqueous phase and sodium dodecyl sulfate, respectively, and stir magnetically to obtain a suspension;
S2. Perform several times of homogenization to the suspension through a microfluidic device to obtain a nanocrystals suspension;
S3. Freeze-dry to obtain Sirolimus nanocrystals;
preferably, the particle size d₅₀ of the nanocrystals particles is 150 to 900 nm, the microfluidization pressure is 1,000 to 2,000 bar, the homogenization is performed for 5 to 10 times, and the mass ratio of Sirolimus to sodium dodecyl sulfate is 5:1 to 1:2.

9. A coating solution for drug-coated balloon, **characterized in that** the coating solution comprises an aqueous solvent, and the coating solution further comprises a plurality of core-shell structures formed by wrapping drugs in a phospholipid bilayer, the core-shell structures are dispersed in the solvent in aqueous phase, wherein, a core of the core-shell structures comprises a plurality of particles loaded with drugs, and a shell of the core-shell structures is a phospholipid bilayer comprising a hydrophilic group in the outer layer and a hydrophobic group in the inner layer, the plurality of particles loaded with drugs comprise a plurality of nanocrystals particles comprising drugs,
wherein, the raw materials for preparing the nanocrystals particles comprise a surfactant, and the surfactant is selected from one or more of vitamin E polyethylene glycol succinate, Poloxamer 188, Poloxamer 407, Tyloxapol, docusate sodium, polyoxyl 15-hydroxystearate, polyoxyethylene (40) hydrogenated castor oil, polyoxyethylene (60) hydrogenated castor oil, polyoxyethylene (60) castor oil, polyoxyethylene (40) castor oil, polyoxyethylene (35) castor oil, polyoxyethylene (20) castor oil, polyoxyethylene (10) castor oil, Polyethylene glycol hexadecyl octadecyl ether 20, polyethylene glycol hexadecyl octadecyl ether 12, polyoxyethylene monocetyl ether (Ceteth-10), polyoxyethylene (10) lauryl ether (Brij 56), polyoxyethylene (20) lauryl ether (Brij 58), polyoxyethylene (23) lauryl ether (Brij 35), polyoxyethylene (2) lauryl ether (Brij 52), polyoxyethylene (25) oleate (Myrj 49), polyoxyethylene (40) palmitate (Myrj 52), sodium dodecyl sulfate, Tween 80, Tween 60, Tween 40, propylene glycol monolaurate, polyoxyethylene (20) lauryl ether, and polyoxyethylene monocetyl ether.

10. A coating solution according to claim 9, **characterized in that** the method for preparing the nanocrystals particles comprises: anti-solvent method, high pressure homogenization method or microfluidic method;
preferably, the anti-solvent method comprises the following steps:
S1. Accurately weigh Sirolimus and organic solvent, respectively, and stir magnetically until a transparent and uniform phase A solution is obtained;
S2. Accurately weigh the surfactant and the solvent in aqueous phase, respectively, and stir magnetically until a transparent and uniform phase B solution is obtained;
S3. Add phase A solution to phase B solution quickly, and stir magnetically to obtain a nanocrystals suspension;
S4. Add water to the suspension and mix well, and then freeze-dry to obtain nanocrystals particles;
preferably, the particle size d₅₀ of the nanocrystals particles is between 150 and 900 nm, and the mass ratio of Sirolimus to surfactant is between 5:1 and 1:2;
preferably, the high pressure homogenization method comprises the following steps:
S1. Accurately weigh Sirolimus, solvent in aqueous phase and surfactant, respectively, and stir magnetically to obtain a suspension;
S2. Perform several times of homogenization to the suspension through a high pressure homogenizer to obtain a nanocrystals suspension;
S3. Freeze-dry to obtain Sirolimus nanocrystals;
preferably, the particle size d₅₀ of the nanocrystals particles is between 150 and 900 nm, the homogenization pressure is 1,000 to 2,000 bar, the homogenization is performed for 5 to 10 times, and the mass ratio of Sirolimus to surfactant is between 5:1 and 1:2;
preferably, the microfluidic method comprises the following steps:
S1. Accurately weigh Sirolimus, solvent in aqueous phase and surfactant, respectively, and stir magnetically to obtain a suspension;
S2. Perform several times of homogenization to the suspension through a microfluidic device to obtain a nanocrystals suspension;
S3. Freeze-dry to obtain Sirolimus nanocrystals;
preferably, the particle size d₅₀ of the nanocrystals particles is 150 to 900 nm, the microfluidization pressure is 1,000 to 2,000 bar, the homogenization is performed for 5 to 10 times, and the mass ratio of Sirolimus to surfactant is 5:1 to 1:2.

11. A use of Sirolimus-coated balloon in intracranial artery stenosis, wherein the drug dose is less than or equal to 3 µg/mm², and more preferably less than or equal to 1.5 µg/mm².
